# EUROPEAN PATENT APPLICATION

(11) **EP 1 176 201 A2**
(43) Date of publication of application: **30.01.2002**
(21) Application number: 01203112.6
(22) Date of filing: 16.05.1989
(51) Int. Cl.: C12N 15/13, C12N 15/62, C07K 16/46, C07K 16/28, A61K 39/395

(54) **Platelet-specific chimeric immunoglobulin**

(30) Priority: 18.05.1988 US 195720
(62) Divisional of application: 89907420.7
(71) Applicant: CENTOCOR, INC., Malvern, PA 19355 (US)
(72) Inventor: Coller, Barry S., Dix Hills, NY 11746 (US)
(74) Representative: Lock, Graham James

(57) **Abstract**

Platelet-specific, chimeric immunoglobulin and immunoglobulin fragments are described. The chimeric molecules are made up of a nonhuman antigen binding region and a human constant region. Preferred immunoglobulins are specific for glycoprotein Iib/IIIa receptor in its complexed form; they block ligand binding to the receptor and prevent platelet aggregation. The immunoglobulins are useful in anti-thrombotic therapy alone or in conjunction with thrombolytic agents and in thrombus imaging.

## Description

### Background of the Invention

Platelet aggregation is an essential event in the formation of blood clots. Under normal circumstances, blood clots serve to prevent the escape of blood cells from the vascular system. However, during certain disease states (i.e. myocardial infarction), clots can restrict or totally occlude blood flow resulting in cellular necrosis.

Heart attack patients are typically treated with thrombolytic agents such as tissue plasminogen activator or streptokinase, which dissolve the fibrin component of clots. A major complication associated with fibrinolysis is reocclusion based on platelet aggregation which can result in further heart damage. Since glycoprotein (gp)IIb/IIIa receptors are known to be responsible for platelet aggregation, reagents which block these receptors are expected to reduce or prevent reocclusion following thrombolytic therapy and to accelerate the rate of thrombolysis.

One approach to blocking platelet aggregation involves monoclonal antibodies specific for gpIIb/ IIIa receptors. A murine monoclonal antibody, designated 7E3, that inhibits platelet aggregation and appears useful in the treatment of human thrombotic diseases is described in published European Patent Application Nos. 205,270 and 206,532. It is known in the art that murine antibodies have characteristics which may severely limit their use in human therapy. As foreign proteins, murine antibodies may elicit immune reactions that reduce or destroy their therapeutic efficacy and/or evoke allergic or hypersensitivity reactions in patients. The need for readministration of such therapeutic modalities in thromboembolic disorders increases the likelihood of these types of immune reactions.

Chimeric antibodies consisting of non-human binding regions joined to human constant regions have been suggested as a means to circumvent the immunoreactivity problems of murine antibodies. See PNAS, 81:6851 (1984) and PCT Application No. PCT/GB85 00392. Since the constant region is largely responsible for immunoreactivity of an antibody molecule, chimeric antibodies with constant regions of human origin should be less likely to evoke an anti-murine response in humans. However, - it is unpredictable whether the joining of a human constant region to a murine binding region of a desired specificity will reduce immunoreactivity and/or alter the binding capability of the resulting chimeric antibody.

### Summary Of The Invention

This invention pertains to a platelet-specific chimeric immunoglobulin comprising a variable or antigen binding region of non-human origin specific and a constant region of human origin. The chimeric immunoglobulins can be specific for gpIIb/IIIa receptor or other platelet components. These antibodies bind to platelets and can block platelet aggregation and thus are useful as antithrombotic agents and to prevent or reduce reocclusion following thrombolysis.

### Brief Description of the Figures

Figure 1 shows a Northern analysis of heavy chain and light chain mRNA's for the 7E3 monoclonal antibody using cloned variable regions as probes.

Figure 2 is a schematic representation of the plasmids p7E3VⱼhCⱼ and p7E3V_{H}hC_{G4}, which carry the chimeric gene constructs for the light and heavy chains, respectively, of the chimeric 7E3 immunoglobulin.

Figure 3 shows the binding of the chimeric 7E3 immunoglobulin to platelets.

Figure 4 shows the inhibition of platelet aggregation by the chimeric 7E3 immunoglobulin.

### Detailed Description Of The Invention

The chimeric immunoglobulins of the invention are comprised of individual chimeric heavy and light immunoglobulin chains. The chimeric heavy chain is comprised of an antigen-binding region derived from the heavy chain of a non-human antibody specific for gpIIb/IIIa receptor linked to a human heavy chain constant region. The chimeric light chain comprises an antigen binding region derived from the light chain of the non-human antibody linked to a human light chain constant region.

The present immunoglobulins can be monovalent, divalent or polyvalent. Monovalent immunoglobulins are dimers (HL) formed of a chimeric heavy chain associated through disulfide bridges with a chimeric light chain. Divalent immunoglobulins are tetramers (H₂L₂) formed of two dimers associated through at least one disulfide bridge. Polyvalent immunoglobulins can also be produced, for example, by employing heavy chain constant region that aggregate (i.g. IgM heavy chains). Chimeric immunoglobulin fragments such as Fab, Fab' or F(ab')₂ can also be produced.

The non-human antigen binding regions of the chimeric immunoglobulin are derived from immunoglobulins specific for platelets. Preferred immunoglobulins are specific for platelet gpIIb/IIIa receptors and block ligand binding to the glycoprotein IIb/IIIa receptor complex. Examples of suitable antibodies include 7E3 and 10E5. See European Patent Application Nos. 205,270 and 206,532, the teachings of which are incorporated herein. The 7E3 antibody (or antibody reactive with the same or a functionally equivalent epitope) is especially preferred because it is specific for the the complexed form of gpIIb/IIIa receptor. Other antibodies are specific for either the IIb or IIIa components can also be used. Antibodies specific for other platelet antigens can be employed. For example, antibodies reactive with platelet a granule membrane protein GMP-140 such as S12 antibody (J. Biol. Chem. 259:9799-9804 (1984)) can be used.

Preferably, the antigen binding region will be of murine origin because murine antibodies against platelets and particularly, gpIIb/IIIa receptors, are available or can be produced in murine systems. Other animal or rodent species provide alternative sources of antigen binding regions.

The constant regions of the chimeric antibodies are derived from human immunoglobulins. The heavy chain constant region can be selected from any of the five isotypes alpha, delta, epsilon, gamma or mu. Further, heavy chains of various subclasses (such as the IgG subclasses of heavy chains) are responsible for different effector functions and thus, by choosing the desired heavy chain constant region, chimeric antibodies with desired effector function can be produced. Preferred constant regions are gamma 1 (IgG1), gamma 3 (IgG3) and gamma 4 (IgG4). The light chain constant region can be of the kappa or lambda type.

In general, the chimeric antibodies are produced by preparing, for each of the light and heavy chain components of the chimeric immunoglobulin, a fused gene comprising a first DNA segment that encodes at least the functional portion of the platelet-specific variable region of nonhuman origin linked (e.g. functionally rearranged variable region with joining segment) to a second DNA segment encoding at least a part of a human constant region. Each fused gene is assembled in or inserted into an expression vector. Recipient cells capable of expressing the gene products are then transfected with the genes. The transfected recipient cells are cultured under conditions that permit expression of the incorporated genes and the expressed immunoglobulins or immunoglobulin chains are recovered.

Genes encoding the variable region of Ig light and heavy chains can be obtained from lymphoid cells that produce the platelet-specific antibodies. For example, the hybridoma cell lines that produce antibody against the gpIIb/IIIa receptor provide a source of immunoglobulin variable region for the present chimeric antibodies. Other rodent cell lines are available. Cell lines can be produced by challenging a rodent with a human platelet or a gpIIb/IIIa receptor-containing component or fraction of platelet, forming fused hybrid cells between antibody-producing cells and a myeloma cell line, cloning the hybrid and selecting clones that produce antibody against platelet or glycoprotein IIb/IIIa receptor.

Constant regions can be obtained from human antibody-producing cells by standard cloning techniques. Alternatively, because genes representing the two classes of light chains and the five classes of heavy chains have been cloned, constant regions of human origin are readily available from these clones. Chimeric antibody binding fragments such as F(ab')₂ and Fab fragments can be prepared by designing a chimeric heavy chain gene in truncated form. For example, a chimeric gene encoding a F(ab')₂ heavy chain portion would include DNA sequences encoding the CH₁ domain and hinge region of the heavy chain.

Preferably, the fused genes encoding the light and heavy chimeric chains (or portions thereof) are assembled in two different expression vectors that can be used to cotransfect a recipient cell. Each vector contains two selectable genes--one for selection in a bacterial system and one for selection in a eukaryotic system--each vector having a different pair of genes. These vectors allow production and amplification of the fused genes in bacterial systems, and subsequent cotransfection of eukaryotic cells and selection of the cotransfected cells. Examples of selectable genes for the bacterial system are the genes that confer ampicillin resistance and the gene that confers chloramphenicol resistance. Two selectable genes for selection of eukarytoic transfectants are preferred: (i) the xanthine-guanine phosphoribosyltransferase gene (gpt), and (ii) the phosphotransferase gene from Tn5 (designated neo). Selection with gpt is based on the ability of the enzyme encoded by this gene to use xanthine as a substrate for purine nucleotide synthesis; the analogous endogenous enzyme cannot. In a medium containing xanthine and mycophenolic acid, which blocks the conversion of inosine monophosphate to xanthine monophosphate, only cells expressing the gpt gene can survive. The product of the neo blocks the inhibition of protein synthesis in eukarytoic cells caused by the antibiotic G418 and other antibiotics of its class. The two selection procedures can be used simultaneously or sequentially to select for the expression of immunoglobulin chain genes introduced on two different DNA vectors into a eukaryotic cell.

The preferred recipient cell line is a myeloma cell. Myeloma cells can synthesize, assemble and secrete immunoglobulins encoded by transfected Ig genes. Further, they possess the mechanism for glycosylation of the immunoglobulin. A particularly preferred recipient cell is the Ig-non-producing myeloma cell Sp2/0. The cell produces only immunoglobulin encoded by the transfected immunoglobulin genes. Myeloma cells can be grown in culture or in the peritoneum of mice where secreted immunoglobulin can be obtained from ascites fluid. Other lymphoid cells such as B lymphocytes or hybridoma cells can serve as suitable recipient cells.

Several methods exist for transfecting lymphoid cell with vectors containing immunoglobulin encoding genes. A preferred way of introducing DNA into lymphoid cells is by electroporation. In this procedure recipient cells are subjected to an electric pulse in the presence of the DNA to be incorporated. See e.g., Potter et al., PNAS 81:7161 (1984). Another way to introduce DNA is by protoplast fusion. In this method, lysozyme is used to strip cell walls from bacteria harboring the recombinant plasmid containing the chimeric Ig gene. The resulting spheroplasts are fused with myelome cells with polyethylene glycol. After protoplast fusion, the transfectants are selected and isolated. Another technique that can be used to introduce DNA into many cell types is calcium phosphate precipitation.

The chimeric immunoglobulin genes can be expressed in nonlymphoid cells such as bacteria or yeast. When expressed in bacteria, the immunoglobulin heavy chains and light chains become part of inclusion bodies. Thus, the chains must be isolated and purified and then assembled into functioned immunoglobulin molecules.

The chimeric platelet-specific antibodies of this invention are useful as antithrombotic therapeutic agents. The chimeric antibodies (or fragments thereof) can be used to inhibit platelet aggregation and thrombus formation. The antibodies can be used in any situation where thrombus formation or reformation is to be prevented. For example, the antibody alone can be used to prevent clotting in post-angioplasty treatment, pulmonary embolism, deep vein thrombosis and coronary bypass surgery. The antibody can also be administered in conjunction with a thrombolytic agent such as tissue plasminogen activator, urokinase, or streptokinase to prevent or reduce reocculusion that can occur after thrombolysis and to accelerate clot lysis. The antibody or fragment can be administered before, along with or subsequent to administration of the thrombolytic agent, in amounts sufficient to prevent platelet aggregation that can result in reocclusion. The antibody is given parenterally, preferably intravenously, in a pharmaceutically acceptable vehicle such as sterile saline. The antibody could be given multiple times or by a controlled release mechanism (e.g. by a polymer or patch delivery system).

During repeat therapy with anti-platelet antibodies drug-induced thrombocytopenia may occur; this may be a result of the body recognizing the antibody-coated platelets as foreign proteins raising antibodies against them and then clearing them more rapidly than normal. The use of a chimeric anti-platelet (e.g. gpIIb/IIIa) antibody may avoid this problem. It is predicted that the majority of the murine component of the chimeric antibody will be bound to the platelet (e.g. the gpIIb/IIIa receptor) and thus not be accessible to the immune system rendering the chimeric antibody functionally indistinguishable from a human antibody directed against the same epitope, and therefore non-immunogenic.

The platelet-specific chimeric immunoglobulins of this invention are also useful for thrombus imaging. For this purpose, antibody fragments are generally preferred. As described above, chimeric heavy chain gene can be designed in truncated form to produce a chimeric immunoglobulin fragment (e.g., Fab, Fab', or F(ab')₂) for immunoscintigraphic imaging. These molecules can be labeled either directly or through a coupled chelating agent such as DTPA, with radioisotopes such as ¹³¹Iodine, ¹²⁵Iodine, ^{99m}Technetium or ¹¹¹Indium to produce radioimmunoscintigraphic agents. Alternatively, a radiometal binding (chelating) domain can be engineered into the chimeric antibody site to provide a site for labeling. Thus, a chimeric immunoglobulin can be designed as a protein that has a nonhuman platelet-specific variable region, a human constant region (preferably truncated), and a metal binding - domain derived from a metal binding protein such as metallothionine.

The platelet-specific chimeric immunoglobulin is administered to a patient suspected of having thrombus. After sufficient time to allow the labeled immunoglobulin to localize at the thrombus site, the signal generated by the label is detected by a photoscanning device such as a gamma camera. The detected signal is then converted to an image of the thrombus. The image makes it possible to locate the thrombus in vivo and to devise an appropriate therapeutic strategy.

The invention is further described by the following examples, wherein all parts and percentages are by weight, and degrees are Celcius unless otherwise stated.

### EXEMPLIFICATION

### Example 1. Production of chimeric platelet specific IgG4.

### A. General strategy

The strategy for cloning the variable regions for the heavy and light chain genes from the 7E3 hybridoma was based upon the linkage in the genome between the variable region and the corresponding J (joining) region for functionally rearranged (and expressed) Ig ganes. J region DNA probes can be used to screen genomic libraries to isolate DNA linked to the J regions; DNA in the germline configuration (unrearranged) would also hybridize to J probes but is not linked to a variable region sequence and can be identified by restriction enzyme analysis of the isolated clones.

The cloning strategy, therefore, was to isolate variable regions from rearranged heavy and light chain genes using J_{H} and J_{K} probes. These clones were tested to see if their sequences were expressed in the 7E3 hybridoma by Northern analysis. Those clones that contained expressed sequences were put into expression vectors containing human constant regions and transfected into mouse myeloma cells to determine if an antibody was produced. The antibody from producing cells was then tested for binding specificity and functionality compared to the 7E3 murine antibody.

### B. Materials and Methods

### Heavy Chain genomic library construction.

To isolate the heavy chain variable region gene from the 7E3 hybridoma, a size-selected genomic library was constructed using the phage lambda vector gt10. Southern analysis of Eco RI digested 7E3 DNA using a J_{H} probe revealed a single 3.5 kb band corresponding to a rearranged heavy chain locus. It was likely that this fragment contained the 7E3 heavy chain variable region gene. High molecular weight DNA was isolated from 7E3 hybridoma cells and digested to completion with restriction endonuclease Eco RI. The DNA was then fractionated on a 0.7% agarose gel and the DNA of size range 3-4 kb was isolated directly from the gel. After phenol/chloroform extraction and Sephadex G-50 gel filtration, the 3-4 kb fragments were ligated with lambda gt10 arms (Promega Biotech, Inc) and packaged into phage particles in vitro using Packagene from Promega Biotech. This library was screened directly at a density of approximately 30,000 plaques per 150 mm petri dish using a ³²P-labeled J_{H} probe. Plaque hybridizations were carried out in 5x SSC, 50% formamide, 2X Denhardt's reagent, 200 lg/ml denatured salmon sperm DNA at 42 degrees C for 18-20 hours. Final washes were in 0.5X SSC, 0.1% SDS at 65 degrees. Positive clones were identified after autoradiography.

### Light chain genomic library construction

To isolate the variable region gene for the 7E3 light chain, a genomic library was constructed in the lambda vector EMBL-3. High molecular weight DNA was partially digested with restriction endonuclease Sau3A and size-fractionated on a 10-40% sucrose density gradient. DNA fragments of 18-23kb were ligated with EMBL-3 arms and packaged into phage particles in vitro using Packagene. This library was screened at a density of 30,000 plaques per 150 mm plate using a Jⱼ probe. Hybridization and wash conditions were identical to those used for the heavy chain library.

### DNA Probes

The mouse heavy chain J_{H} probe is a 2 kb BamHI/EcoRI fragment containing both J3 and J4 segments. The mouse light chain Jⱼ probe is a 2.7 kb HindIII fragment containing all five Jⱼ segments. ³²P-labeled probes were prepared by nick translation using a kit obtained from Amersham, Inc. Free nucleotides were removed by centrifugation through a Sephadex G-50 column. The specific activities of the probes were approximately 10⁹ cpm/lg.

### Northern Analysis

15 lg total cellular RNA was subjected to electrophoresis on 1% agarose/formaldehyde gels (Maniatis, et al, Molecular Cloning) and transferred to nitrocellulose. Blots were hybridized with nick translated DNA probes in 50% formamide, 2X Denhardt's solution, 5x SSC, and 200 lg/ml denatured salmon sperm DNA at 42 degrees for 10 hours. Final wash conditions were 0.5X SSC 0.1% SDS at 65 degrees.

### DNA Transfection using Electroporation

Plasmid DNA to be transfected was purified by centrifuging to equilibrium in ethidium bromide/ cesium chloride gradients two times. 10-50 lg of plasmid DNA was added to 8 x 10⁶ SP2/0 cells in PBS on ice and the mixture placed in a Biorad electroporation apparatus. Electroporation was at 200 volts and the cells were plated out in 96 well microtiter plates. Appropriate drug selection was - applied after 48 hours and drug resistant colonies were identified after 1-2 weeks.

### Quantitation of antibody production

Tissue culture supernatant was analyzed for IgG protein content by particle concentration fluorescence immunoassay (Jolley, M.E. et al, (1984) J. Immunol. Meth. 67:21) using standard curves generated with purified IgG. Concentration of chimeric 7E3 antibody with human constant regions was determined using goat antihuman IgG Fc antibody-coated polystyrene beads and fluorescein conjugated goat anti-human IgG Fc antibody. The assay was carried out with an automated instrument (Pandex Laboratories, Inc.)

### Purification of platelet-specific chimeric IgG4 Antibody

Tissue culture supernatant was concentrated with a Diaflo YM100 ultrafiltration membrane (Amicon), and loaded onto a protein A-sepharose column. The chimeric antibody was eluted from the protein A column with a sodium citrate pH gradient from pH 6.5 to pH 3.5. The purified antibody was concentrated using a Diaflo YM100 ultrafiltration membrane. Antibody concentration was measured by determining the absorbance at 280 nm.

### Binding Inhibition Assay

Purified antibody (either murine 7E3 or chimeric 7E3) was used to compete with radioiodinated 7E3 antibody for binding to human platelets.
Platelet-rich plasma (PRP) was prepared by centrifugation of citrated whole human blood at 1875 rpm for 3.5 minutes. ¹²⁵I-labeled 7E3 antibody (150,000 cpm) was added to the appropriate dilution of the purified test antibody and the reaction was initiated by the addition of 150 11 PRP. Incubation was for 1-2 hours at room temperature and the platelets with bound antibody were separated from free antibody by centrifugation through 30% sucrose at 12,000 g for 4 minutes in a 0.4 ml microfuge tube. The tube tip containing the platelet/antibody pellet was cut off and counted in a gamma counter. The competition for binding to platelets between iodinated 7E3 and chimeric 7E3 was compared to the competition between iodinated 7E3 and unlabeled 7E3 IgG.

### Inhibition of Platelet aggregation

Purified 7E3 or chimeric 7E3 antibody was added to citrated whole human blood and incubated at 37 degrees for 10 minutes. The rate of platelet aggregation was measured after activation with collagen or ADP using a whole blood aggregometer (Chronolog Corp.).

### C. Results

### Cloning of the platelet-specific variable gene regions

Several positive clones were isolated from the heavy and light chain libraries after screening approximately one million plaques using the J_{H} and Jⱼ probes, respectively. Following at least three rounds of plaque purification, bacteriophage DNA was isolated for each positive clone, digested with either EcoRI (heavy chain clones) or HindIII (light chain clones) and fractionated on 1% agarose gels. The DNA was transferred to nitrocellulose and the blots were hybridized with J_{H} (heavy chain) or Jⱼ ³²P-labeled DNA probes. For the heavy chain, 2 clones were obtained that contained 3.5 kb Eco RI DNA fragments that hybridized to the J_{H} probe. Two size classes of HindIII fragments of 3.0 and 6.0 kb were identified with the Jⱼ probe.

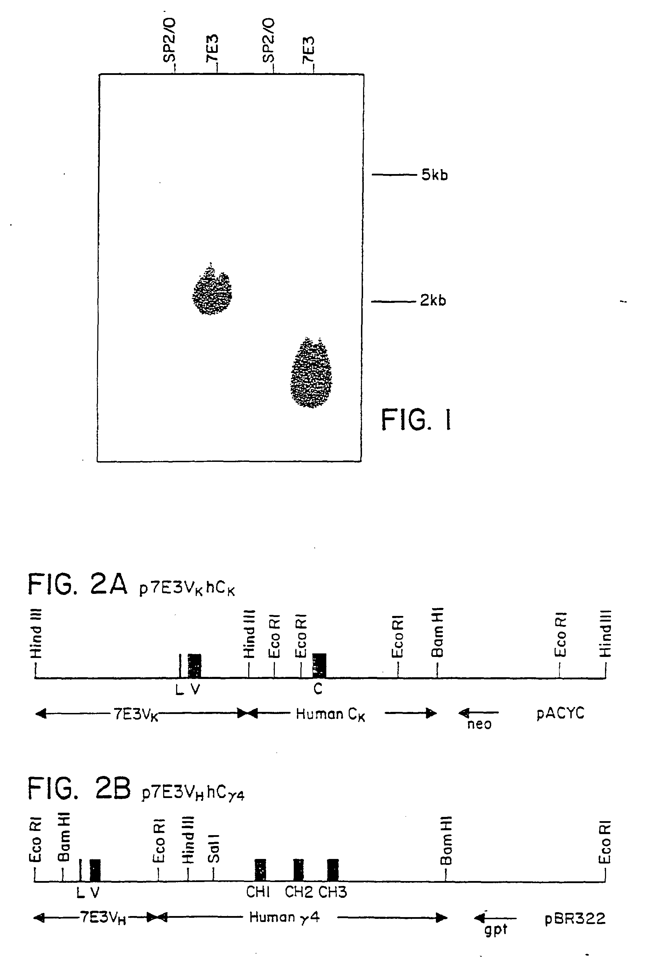

Cloned DNA corresponding to the authentic heavy and light chain variable regions from the 7E3 hybridoma should hybridize to mRNA isolated from the hybridoma. Non-functional DNA rearrangements at either the heavy or light chain loci should not be expressed. Figure 1 shows a Northern analysis that demonstrates that the 3.5 kb EcoRI putative heavy chain fragment and the 6.0 kb HindIII putative light chain fragment each hybridizes to the appropriate size mRNA from the 7E3 hybridoma. The subcloned fragments were labeled with ³²P by nick translation and hybridized to northern blots containing total RNA derived from SP2/0 (the fusion partner of the 7E3 hybridoma) or from 7E3. The 3.5 kb EcoRI heavy chain fragment hybridizes with a 2 kb mRNA in 7E3 RNA but not in SP2/0 RNA. Similarly, the 6.0 kb light chain HindIII fragment hybridizes with a 1250 bp mRNA in 7E3 RNA but not in SP2/0 RNA. These are the correct sizes for heavy and light chain mRNAs respectively. Because the cloned DNA fragments contain sequences expressed in the 7E3 hybridoma, these data suggest that these are the correct variable region sequences from the 7E3 hybridoma. The final functional test, however, is the demonstration that these sequences, when combined with the appropriate constant region sequences, are capable of directing the synthesis of an antibody with a specificity and affinity similar to that of the murine 7E3 antibody.

### Vectors and Expression systems

The putative light and heavy chain V genes cloned from the 7E3 hybridoma were joined to human j and G4 constant region genes in expression vectors described previously (Sun, L. et al., PNAS 84, p. 214-218 (1987). The 17-1A Vⱼ HindIII fragment of pSV184DHneo17-1AVⱼhCⱼ was replaced with the 6.0 kb HindIII fragment corresponding to the putative light chain variable region gene from 7E3. Similarly, the 17-1A V_{H} Eco RI fragment of pSV2DHgpt17-1AV_{H}-hC_{G4} was replaced with the 3.5 kb EcoRI fragment corresponding to the putative heavy chain V region gene from 7E3. The structures of the resulting plasmids, designated p7E3VⱼhC_{Hj} and p7E3V_{H}hC_{G4} , are shown in figure 2.

To express the chimeric heavy and light chain genes, the two plasmids were cotransfected into the nonproducing mouse myeloma cell line SP2/0. The light chain plasmid confers resistance to G418 and - the heavy chain plasmid confers resistance to mycophenolic acid, thus allowing a double selection to be used to obtain clones carrying and expressing genes from each plasmid. Colonies resistant to G418 and mycophenolic acid were expanded to stable cell lines and maintained in the presence of both drugs. Tissue culture supernatant from these cell lines was tested for antibody using a particle concentration fluorescence immunoassay with polystyrene beads coated with goat anti-human IgG Fc antibody and the same antibody labeled with fluorescein. Out of the first 10 lines checked, one (designated c-7E3F6) that produced approximately 21g/ml was selected for further study.

### Platelet binding activity assay

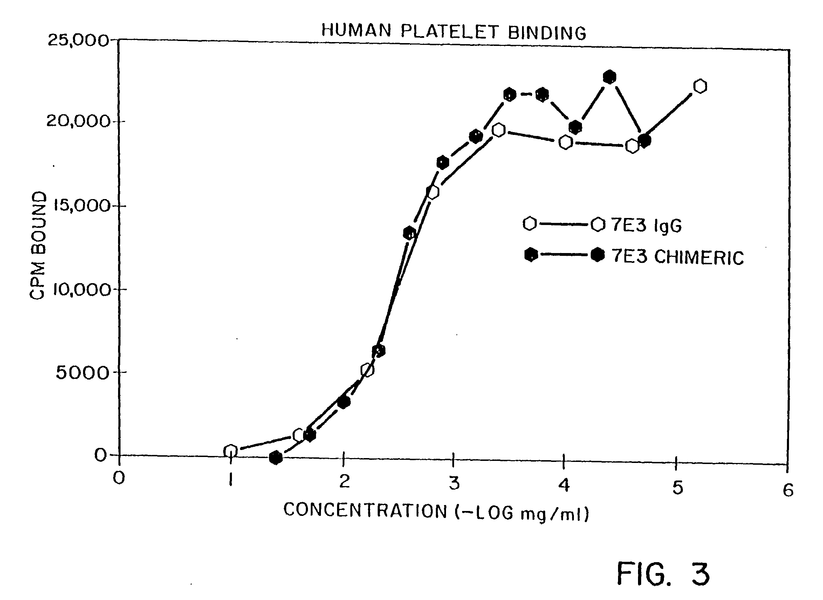

After purification of c-7E3F6 antibody using a protein A-sepharose column, the antibody was concentrated and compared to murine 7E3 IgG in the platelet binding activity assay. Figure 3 shows that murine 7E3 and c-7E3F6 (the putative chimeric antibody) compete with radiolabeled 7E3 for platelet binding to the same extent; the binding curves are superimposable indicating that the binding characteristics of murine and chimeric 7E3 are identical.

### Inhibition of platelet aggregation by C-7E3F6

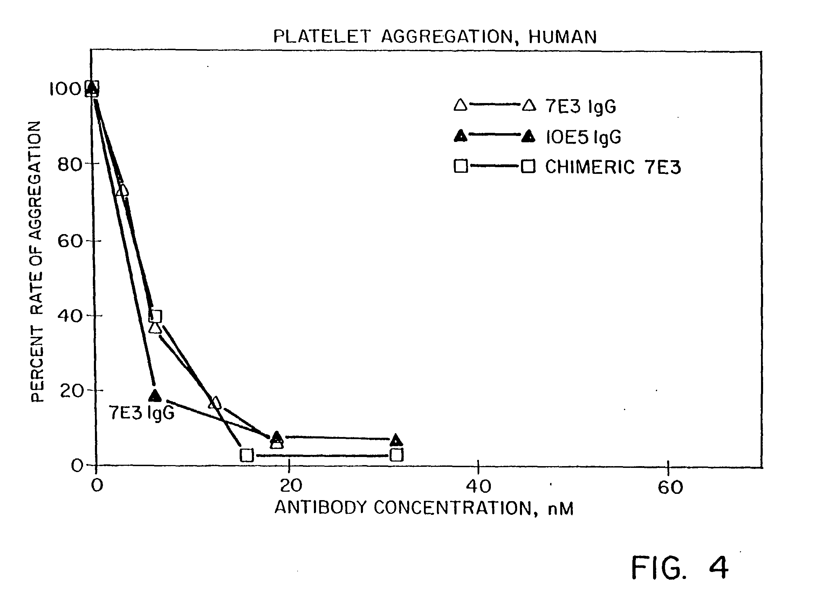

Purified c-7E3F6 was compared to murine 7E3 in a functional assay that measures the ability of the test antibody to inhibit aggregation of human platelets. The results shown in figure 4 demonstrate that both antibodies inhibit collagen-induced platelet aggregation to the same extent at the same antibody concentration. c-7E3F6 also inhibits ADP-induced platelet aggregation to a similar extent.

The results of the platelet binding assay and the inhibition of platelet aggregation assay demonstrate 1.) that the correct variable region genes were indeed cloned from the 7E3 hybridoma; 2.) that substitution of the human constant regions for the mouse constant regions has no effect on the binding or functional characteristics of the 7E3 variable regions as measured by these assay.

### Fibrogen-coated bead assay

The chimeric c-7E3F6 antibody was found positive in a qualitative, functional assay that measures the ability of an antibody to inhibit the agglutination between platelets and fibrinogen-coated beads. Coller, B. et al. (1983) J. Clin. Invest. 73:325-338.

### Example 2. Production of chimeric IgG1 and IgG3.

The DNA segment encoding the variable region of the heavy chain from the murine 7E3 antibody was linked to the human 1 and 3 constant regions present on the expression vectors pSV2DHgpt17-1AV_{H}-hC_{G1} and pSV2DHgpt17-1AV_{H}-hC_{G3} (Sun, et al., PNAS 84, p. 214-218, 1987) by replacing the 17-1A variable region fragments with the 7E3 variable region fragment. The resulting chimeric heavy chain genes were cotransfected with the chimeric light chain gene into SP2/0 cells to generate stable cell lines secreting c1,K, and c3,K antibodies.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. A platelet-specific chimeric immunoglobulin comprising an antigen binding region of non-human origin and a constant region of human origin.

2. A chimeric immunoglobulin of Claim 1, wherein the antigen binding region is derived from a murine anti-platelet immunoglobulin.

3. A chimeric immunoglobulin of Claim 1 which is specific for glycoprotein IIb/IIIa receptor complex and blocks the binding of ligand thereto.

4. A chimeric immunoglobulin of Claim 3, wherein the antigen binding region is derived from the monoclonal antibody 7E3.

5. An antigen binding fragment of a chimeric immunoglobulin of Claim 1.

6. A radiolabeled antigen binding fragment of Claim 5.

7. A chimeric immunoglobulin comprising:
a. at least one chimeric heavy chain comprising an antigen binding region derived from the heavy chain of a nonhuman immunoglobulin specific for glycoprotein IIa/IIIb receptor linked to at least a portion of a human heavy chain constant region, the heavy chain being in association with:
b. at least one chimeric light chain comprising an antigen binding region derived from a light chain of the nonhuman immunoglobulin linked to at least a portion of a human light chain constant region.

8. A chimeric immunoglobulin of Claim 7, wherein the antigen binding region is derived from a murine antibody.

9. A chimeric immunoglobulin of Claim 8, wherein the antigen binding region is derived from the monoclonal antibody 7E3.

10. A chimeric immunoglobulin fragment Fab, Fab' or F(ab')₂ comprising a murine variable region specific for the glycoprotein IIb/IIIa receptor complex and a human constant region.

11. A chimeric immunoglobulin fragment of Claim 10, wherein the variable region is derived from the monoclonal antibody 7E3.

12. A chimeric immunoglobulin fragment of Claim 10, which is radiolabeled.

13. A chimeric immunoglobulin fragment of Claim 12, wherein the radiolabel is ^{99m}Tc or ¹¹¹In.

14. A fused gene encoding a chimeric light or heavy chain immunoglobulin comprising:
a. a first DNA sequence encoding an immunoglobulin variable region of a platelet-specific antibody of nonhuman origin linked to:
b. a second DNA sequence encoding a constant region of an immunoglobulin of human origin.

15. A fused gene of Claim 14, wherein the variable region of the immunoglobulin chain is of murine origin.

16. A fused gene of Claim 15, wherein the variable region is derived from the monoclonal antibody 7E3.

17. An expression vector containing the fused gene of Claim 14 in expressible form.

18. A method of antithrombotic therapy, comprising administering to a patient having a thrombus or at risk of thrombus formation, an antithrombotic amount of a chimeric immunoglobulin or immunoglobulin fragment comprising an antigen binding region of nonhuman origin specific for platelet and a human constant region.

19. A method of Claim 8, wherein the antigen binding region of nonhuman origin is specific for glycoprotein IIb/IIIa.

20. A method of Claim 19, wherein the antigen binding region is derived from the monoclonal antibody 7E3.

21. A method of Claim 18, wherein the immunoglobulin fragment is an Fab, Fab' or F(ab')₂ fragment.

22. A method of antithrombotic therapy, comprising administering to a patient having a thrombus or at risk or thrombus formation, a thrombolytic agent and a chimeric immunoglobulin or immunoglobulin fragment comprising an antigen binding region of nonhuman origin specific for platelet and a human constant region.

23. A method of Claim 22, wherein the chimeric immunoglobulin or immunoglobulin fragment is administered along with or subsequent to administration of the thrombolytic agent.

24. A method of Claim 22, wherein the thrombolytic agent is tissue plasminogen activator, streptokinase, or urokinase.

25. A method of Claim 22, wherein the antigen binding region of nonhuman origin is specific for glycoprotein IIb/IIIa.

26. A method of Claim 25, wherein the antigen binding region is derived from the monoclonal antibody 7E3.

27. A method of Claim 22, wherein the immunoglobulin fragment is an Fab, Fab' or F(ab')₂ fragment.

28. A method of thrombus imaging comprising:
a. administering to an individual suspected of having a thrombus, a radiolabeled chimeric platelet-specific immunoglobulin or fragment thereof, comprising an antigen binding region of nonhuman origin and a constant region of human origin;
b. allowing the antibody or antibody-fragment to accumulate at a thrombus site;
c. detecting the signal generated by the radiolabel by means of a photoscanning device; and
d. converting the detected signal to an image of the thrombus.

29. A method of Claim 28, wherein a Fab, Fab' or F(ab')₂ fragment is administered.

30. A method of Claim 28, wherein the radiolabel is ^{99m}Tc or ¹¹¹In.

31. A method of Claim 28, wherein the platelet-specific immunoglobulin is specific for glycoprotein IIb/IIIa receptor.

32. A method of Claim 31, wherein antigen binding region is derived from the monoclonal antibody 7E3.
